# EUROPÄISCHE PATENTSCHRIFT

(11) **EP 3 328 357 B1**
(45) Veröffentlichungstag und Bekanntmachung des Hinweises auf die Patenterteilung: **09.12.2020**
(21) Anmeldenummer: 16759665.9
(22) Anmeldetag: 25.07.2016
(51) Int. Cl.: A61K 31/5575, A61P 7/00, A61K 9/00, A61K 47/10, A61K 9/08

(54) **KONZENTRAT ENTHALTEND ALPROSTADIL**
CONCENTRATE CONTAINING ALPROSTADIL
CONCENTRÉ CONTENANT DE L'ALPROSTADIL

(30) Priorität: 27.07.2015 AT 506642015
(43) Veröffentlichungstag der Anmeldung: 06.06.2018
(73) Patentinhaber: Gebro Holding GmbH, 6391 Fieberbrunn (AT)
(72) Erfinder: HESSE, Ernst, 6391 Fieberbrunn (AT); HANSBAUER, Bernhard, 5061 Elsbethen-Glasenbach (AT); HÄUSLER, Franz, 83435 Bad Reichenhall (DE)
(74) Vertreter: Wildhack & Jellinek
(86) Internationale Anmeldenummer: PCT/AT2016/060019
(87) Internationale Veröffentlichungsnummer: WO 2017/015691

(56) Entgegenhaltungen:
- US-A- 5 681 855
- HEIDER P ET AL: "Improvement of microcirculation after percutaneous transluminal angioplasty in the lower limb with Prostaglandin E1", PROSTAGLANDINS AND OTHER LIPID MEDIATORS, ELSEVIER, US, Bd. 88, Nr. 1-2, 1. Januar 2009 (2009-01-01), Seiten 23-30, XP025815665, ISSN: 1098-8823, DOI: 10.1016/J.PROSTAGLANDINS.2008.09.001 [gefunden am 2008-09-11]
- "Pridax Konzentrat zur Herstellung einer Infusionslösung", , 1. August 2013 (2013-08-01), XP055196343, Gefunden im Internet: URL:http://www.pharmore.de/pdf_files/pharm ore-plus/pridax_fi_aug_2013.pdf [gefunden am 2015-06-17]

## Beschreibung

Die Erfindung betrifft eine flüssige pharmazeutische Zusammensetzung in Form eines Konzentrats zur Herstellung einer Infusionslösung, enthaltend, insbesondere bestehend aus, Alprostadil (Prostaglandin E1) als Wirkstoff, gelöst in einem pharmazeutisch annehmbaren organischen Lösungsmittel oder in einem Lösungsmittelgemisch aus zumindest einem pharmazeutisch annehmbaren organischen Lösungsmittel und Wasser, zur Verwendung in der Behandlung der peripheren arteriellen Verschlusskrankheit (pAVK).

Bei der peripheren arteriellen Verschlusskrankheit (pAVK) handelt es sich um eine Störung der arteriellen Durchblutung der Extremitäten. Die Krankheit stellt eine im Wesentlichen durch eine Arteriosklerose verursachte, chronische Durchblutungsstörung der Arterien dar, die sich meistens an den unteren Extremitäten manifestiert. Sie entsteht durch Einengung (Stenose) oder Verschluss (Okklusion) der die Extremitäten versorgenden Arterien. Die periphere arteriellen Verschlusskrankheit ist eine häufige Erkrankung, deren Prävalenz mit dem Alter stark zunimmt. Allein in Deutschland leiden nach Angaben der Deutschen Gesellschaft für Angiologie - Gesellschaft für Gefäßmedizin rund 4,5 Millionen Menschen an einer pAVK. Sie ist für die Betroffenen mit schwerwiegenden Beeinträchtigungen, wie Schmerz und Einschränkung der Mobilität, verbunden und verursacht hohe gesellschaftliche Kosten durch Krankenhausaufenthalte, Arbeitsunfähigkeit und Frühverrentung. Für das Auftreten der Erkrankung gibt es eine Reihe äußerer und genetischer Risikofaktoren und die Erkrankung gilt insbesondere als Folgeerkrankung des inhalativen Rauchens und des Diabetes mellitus.

Die Entwicklung der peripheren arteriellen Verschlusskrankheit verläuft langsam und schleichend und wird in den meisten Fällen erst durch das Auftreten des typischen Beschwerdebildes erkannt. Dementsprechend wird die Erkrankung auch entsprechend ihrem Beschwerdebild nach Fontaine in vier Stadien eingeteilt.

**Tabelle 1: pAVK Stadien nach Fontaine**

| Stadium | Symptomatik |
|---|---|
| Fontaine I | Minderdurchblutung ohne Symptomatik |
| Fontaine II | Claudicatio intermittens |
| Fontaine III | Ruheschmerz |
| Fontaine IV | Trockene oder feuchte Gangrän |

Die Claudicatio intermittens zeigt sich durch Schmerzen in den Beinen, die unter Belastung, z.B. beim Gehen auftreten, und die Patienten zum Stehenbleiben zwingen. Nach einer gewissen Ruhepause geht die Schmerzempfindung zurück und die Betroffenen können eine gewisse Strecke schmerzfrei weitergehen. Die leichterte Form dieser Symptomatik wird auch als Stadium Fontaine II a bezeichnet und zeichnet sich durch ein beschwerdefreie Gehstrecke von mehr als 200 m aus; bei der schwereren Form, Fontaine Stadium II b, ist die beschwerdefreie Gehstrecke kürzer als 200 m.

Beim Stadium III nach Fontaine liegt eine schwere, schlecht kompensierte Durchblutungsstörung vor, die durch zahlreiche, in unterschiedlicher Höhe lokalisierte arterielle Verschlüsse und Verengungen verursacht wird. Die Patienten leiden unter ständigen Schmerzen, die nicht auf eine bestimmte Region beschränkt sind. Meist sind die kleinen Arterien an den Füssen besonders betroffen, sodass insbesondere die Zehen und die Fußsohle schmerzhaft verändert sind und auch dort bevorzugt weitere Komplikationen auftreten.

Das Stadium IV nach Fontaine ist durch mehr oder weniger ausgeprägte nekrotische Ulzera geprägt, die zum irreversiblen Gewebsverlust führen. Geschwüre treten insbesondere am Unterschenkel, am Knöchel und an den Füssen, einschließlich der Zehen auf. Dabei kann es sich um einen trockenen Gewebsuntergang handelt, der mit einer Schwarzfärbung des Gewebes einhergeht, oder es treten nässende Gangränen auf, die häufig massiv bakteriell infiziert sind. Ohne antibiotische Behandlung können diese Infektionen zu einer lebensbedrohlichen Sepsis führen. In einem unbeherrschbaren Stadium IV der pAVK ist die Amputation der betroffenen Gliedmaßen die letzte therapeutische Alternative.

Die Prophylaxe und die Therapie der pAVK umfasst in allen Stadien und als Basisbehandlung die Elimination der wesentlichen Risikofaktoren, also das Einstellen des Rauchens, die Reduktion von Übergewicht und die optimale Einstellung des Blutdrucks und allenfalls bestehender Stoffwechselerkrankungen, insbesondere des Diabetes mellitus. Dazu kommen in den Stadien I und II und in den höheren Stadien, soweit das möglich ist, physikalische und physiotherapeutische Behandlungsmaßnahmen. Die Möglichkeiten, den Krankheitsverlauf medikamentös günstig zu beeinflussen, sind begrenzt. Eingesetzt werden Medikamente, die die Blutrheologie verbessern und einer Thrombose vorbeugen, z. B. Antikoagulantia oder Mittel zur Fibrinogen Senkung.

Der einzige direkt kausal wirkende, medikamentöse Therapieansatz ist die Gabe von gefäßerweiternden Substanzen, die den Gefäßwiderstand reduzieren und die Arterien dilatieren. Verwendet werden dazu fast ausschließlich Abkömmlinge des Prostazyklins, insbesondere die Prostaglandine PGI2 (Epoprostenol) und PGE1 (Alprostadil). Beide Substanzen bewirken eine starke Dilatation der Arterien und wirken gleichzeitig als potente Thrombozytenaggregationshemmer. Beide Wirkqualitäten sind bei der Behandlung der pAVK erwünscht. Prostaglandine sind mit Einschränkung im Stadium II b und klar in den Stadien III und IV indiziert.

Alprostadil (PGE1) ist ein im Europäischen Arzneibuch beschriebener Wirkstoff aus der Gruppe der Prostaglandin Analoga. Es ist identisch mit dem körpereigenen Prostaglandin E1. Seine hauptsächlichen physiologischen Effekte bestehen in einer Vasodilatation und in einer Hemmung der Thromozytenaggregation. Aus diesem Wirkprofil leiten sich als die wichtigsten Indikationen die Behandlung erektiler Dysfunktion, die Behandlung peripherer arterieller Verschlusskrankheiten und die kurzzeitige Behandlung bestimmter Herzfehler beim Neugeborenen ab. Als pharmazeutische Darreichungsformen stehen dafür hauptsächlich parenterale, zur Behandlung der erektilen Dysfunktion auch topische Arzneiformen zur Verfügung.

Alprostadil ist ein chemisch außerordentlich instabiler Wirkstoff, der insbesondere unter Abspaltung von Wasser zu Prostaglandin A₁ (PGA1) abgebaut wird. Diese Reaktion wird durch die Anwesenheit von Säuren oder Basen noch beschleunigt; eine orale Gabe von Alprostadil ist deshalb nicht möglich.

Nach parenteraler Gabe erfährt Alprostadil eine rasche Metabolisierung und Verteilung. Es wird an Serumalbumin und andere Proteinfraktionen gebunden und in die verschiedenen Gewebe verteilt. Der Metabolismus verläuft im ersten Schritt oxidativ und findet hauptsächlich in der Lunge statt. Alle Metaboliten sind weniger wirksam als das Alprostadil selbst. Die Ausscheidung erfolgt hauptsächlich über die Niere. Die Eliminationshalbwertszeit beträgt nur wenige Minuten. Die therapeutische Verwendung erfolgt daher - mit Ausnahme in der Behandlung erektiler Dysfunktion - ausschließlich in Form von Dauerinfusionen.

Parenterale Zubereitungen, die direkt in die Blutbahn gelangen sollen, werden in den meisten Fällen intravenös (i.v.) injiziert oder infundiert. Diese Form der Applikation ist für die Patienten die angenehmste und die Gefahr von Komplikationen, insbesondere von schwer stillbaren Blutungen ist gering. Bei der i.v. Applikation wird der Wirkstoff durch das seitlich aus den anderen Venen zuströmende Blut rasch verdünnt, was die lokale Verträglichkeit an den Epithelien verbessert. Über den Blutkreislauf wird der Wirkstoff relativ rasch gleichmäßig im gesamten Plasmavolumen verteilt und es findet von dort aus die wirkstofftypische Verteilung in die anderen Gewebe und Kompartimente statt.

Im Fall der Verwendung von Alprostadil zur Behandlung der peripheren arteriellen Verschlusskrankheit liegt allerdings in zweifacher Hinsicht ein Sonderfall vor, der die i.v. Applikation als nachteilig und stattdessen eine intraarterielle (i.a.) Applikation vorteilhaft erscheinen lässt:
Zum einen stellt in dieser Indikation gerade das arterielle Gefäßsystem den Wirkort für den Arzneistoff dar und eine hohe Wirkstoffkonzentration in den Arterien, insbesondere in den arteriellen Endgefäßen der unteren Extremitäten ist gewünscht.

Zum anderen geht bei der i.v. Applikation im Fall des Alprostadils wegen der sehr kurzen Plasmahalbwertszeit des Stoffes auch während des an sich kurzen Zeitraums bis zur gleichmäßigen Plasmaverteilung bereits ein erheblicher Teil des Wirkstoffes metabolisch verloren.

Durch die intravenöse (i.v.) Verabreichung von Alprostadil kann daher die notwendige hohe Wirkstoffkonzentration speziell und direkt am Wirkort, nämlich den Arterien der unteren Extremitäten, oft nicht im gewünschten Ausmaß erreicht werden und therapeutisch wirksame Konzentrationen lassen sich nur bei einer i.a. Verabreichung erreichen.

Alprostadil zur parenteralen Anwendung wird wegen der Instabilität des Wirkstoffes ausschließlich in Form von Konzentraten angeboten, die kurz vor der Anwendung durch Auflösung in einer geeigneten sterilen Lösung (z.B. physiologische Kochsalzlösung) zur gebrauchsfertigen Infusionslösung verdünnt bzw. rekonstituiert werden. Als Konzentrate werden derzeit im Wesentlichen entweder nicht-wässrige, speziell ethanolische Lösungen oder Lyophilisate verwendet.

Nicht-wässriges, speziell ethanolisches Alprostadil-Konzentrat:
Als Alprostadil-Konzentrate zur Herstellung von Infusionslösungen in Form nicht-wässriger Lösungen werden im Wesentlichen Lösungen des Wirkstoffes in absolutem Ethanol verwendet. So ist aus dem Stand der Technik Alprostadil zur Behandlung der arteriellen Verschlusskrankheit bereits in Form eines ethanolischen Konzentrats bekannt. Handelsbezeichnungen solcher Produkte sind z.B. Pridax® (Gebro, Pharmore) oder Alprestil® (Gebro). Die einzige für diese und ähnliche Produkte zugelassene Applikationsform ist die intravenöse (i.v.) Verabreichung nach Verdünnung zu einer Infusionslösung. Für die intraarterielle Anwendung sind diese Zubereitungen bisher nicht zugelassen und sie stehen damit für die intraarterielle Verabreichung in der Behandlung der peripheren arteriellen Verschlusskrankheit nicht zur Verfügung.

Eine für die Behandlung der peripheren arteriellen Verschlusskrankheit übliche Dosierung bei der intravenösen (i.v.) Verabreichung einer derartigen ethanolischen Alprostadil-Lösung (Pridax®) ist die Infusion von 20 µg Alprostadil pro Stunde über insgesamt 2 bis 3 Stunden pro Tag. Dies entspricht bei Verwendung von Pridax®-Ampullen einer maximalen Zufuhrmenge von 2,4 g Ethanol pro Tag bzw. einer Zufuhrrate von 0,8 g Ethanol pro Stunde während 2 bis 3 Stunden. Diese Alkoholmenge führt zu Blutalkoholkonzentrationen, die üblicherweise unterhalb von 0,2 ‰ bleiben und die, von besonderen Patientengruppen abgesehen, toxikologisch meist unkritisch sind.

Obwohl auch diese geringe physiologische Belastung mit Ethanol per se unerwünscht ist, gilt sie bei intravenöser Verabreichung als akzeptabel und nur bei bestimmten Patientengruppen, z.B. bei Alkoholabhängigen, als problematisch. Im Gegensatz dazu gibt es jedoch gegenüber der intraarteriellen Anwendung einer alkoholhaltigen Infusionslösung, selbst bei geringen Alkoholgehalten, massive Vorbehalte, da bei dieser Applikationsform wegen der langsamen Verdünnung bzw. Verteilung der Wirkstofflösung im Plasma lokal am Wirkort wesentlich höhere Konzentrationen auftreten. Wegen der unveränderlichen Position des intraarteriellen Eingangs sind auch während der gesamten Infusion bzw. bei wiederholten Infusionen immer wieder die gleichen Gefäßregionen der maximalen Alkoholkonzentration ausgesetzt. Diese maximale Alkoholkonzentration ergibt sich direkt an der Zuflussstelle der Infusion aus der Alkoholkonzentration der Arzneistofflösung selbst, die bis zu 1,6 %, entsprechend 16 ‰ betragen kann.

Bedenken gegen die entstehenden, relativ hohen intraarteriellen Alkoholkonzentrationen bestanden bisher vor allem aus zwei Gründen:
Die erste Befürchtung bestand darin, dass Alkohol selbst auf die arteriellen Gefäße eine gefäßverengende Wirkung hat, die der durch die Medikation angestrebten Gefäßerweiterung entgegengesetzt wäre. Diese Befürchtung einer therapeutischen Inkompatibilität zwischen dem Wirkstoff Alprostadil und dem Hilfsstoff Ethanol konnte allerdings weitgehend beseitigt werden, da gezeigt werden konnte, dass bei intraarterieller Verabreichung Ethanol alleine zwar die Gefäße verengt, dass der Ethanol aber in Kombination mit Vasodilatatoren (Methacholin, Nitroprussid, Verapamil) deren Wirkung sogar verstärkt (A. Tawakol et al., Am J Physiol Heart Circ Physiol 286, H2468-H2473, 2004).

Die zweite Befürchtung bestand darin, dass die relativ hoch konzentrierte ethanolische Lösung eine lokale Unverträglichkeit an und in den Gefäßen in unmittelbarer Nähe des intraarteriellen Eingangs verursacht, einerseits verursacht durch eine Schädigung des empfindlichen, einschichtigen Gefäßendothels, andererseits verursacht durch die Schädigung von Blutzellen, insbesondere von Erythrozyten (Hämolyse) und Thrombozyten (Bildung von Thrombosen).

Lyophilisiertes Alprostadil Konzentrat:
Weiters ist aus dem Stand der Technik die Verwendung eines lyophilisierten, also eines festen, pulverförmigen Alprostadil-Konzentrats, bekannt, z.B. das Produkt Prostavasin®. Dieses wird für den Gebrauch in physiologischer Kochsalzlösung gelöst und die Infusionslösung enthält damit keinen Alkohol bzw. kein organisches Lösungsmittel. Heider et al. beschrieb 2009 (HEIDER P ET AL., "Improvement of microcirculation after percutaneous transluminal angioplasty in the lower limb with Prostaglandin E1 "(2009-01 -01)) eine Verbesserung der Mikrozirkulation bei Patienten mit PAOD (peripherer arterieller Verschlusskrankheit) gemäß Fontane Klassifikation Stadium IIb durch intra-arterielle Gabe von Prostavasin™, lyophilisiertem PGE1 (Alprostadil) gelöst in Kochsalzlösung. In Zusammenhang mit diesem Lyophilisat und seiner Anwendung wurde allerdings kürzlich folgendes festgestellt:
In einer bisher unpublizierten, aber unter clincicaltrials.gov des "U.S. National Institutes of Health" (NCT00596752) einsehbaren und auf der 43. Jahrestagung der deutschen Gesellschaft für Angiologie im September 2014 vorgetragenen Studie (H. Lawall, Ergebnisse einer klinischen Studie mit Prostavasin® bei Patienten mit kritischer Extremitätenischämie) wurde die Wirksamkeit von Alprostadil bei der Behandlung der peripheren arteriellen Verschlusskrankheit im Stadium IV (nach Fontaine) gegen Placebo untersucht. An der Studie nahmen insgesamt 840 Patienten teil, 416 in der Alprostadil Gruppe, 423 in der Placebo Gruppe. Die Medikation wurde in Form einer intravenösen Infusion von 40 µg Alprostadil in isotonischer Kochsalzlösung über 2 Stunden gegeben. Als primäre klinische Endpunkte wurden die vollständige Abheilung von Nekrosen und Ulcera sowie die Zahl der nötig gewordenen Amputationen ausgewertet; darüber hinaus wurden zehn weitere sekundäre klinische Endpunkte bei der Auswertung berücksichtigt. Insgesamt zeigte die Studie keinen statistisch signifikanten Vorteil der Alprostadil Behandlung gegenüber der Placebo Behandlung.
Als Folge dieses Ergebnisses wurde erwogen für die verwendete Zubereitung, Prostavasin® Trockensubstanz zur Infusionsbereitung, die intravenöse Verabreichung beim Stadium IV der chronischen Verschlusskrankheit als empfehlenswerte Applikationsform zu streichen. Nach dem Stand des Wissens sollte demnach Prostavasin® zur Behandlung der arteriellen Verschlusskrankheit im Stadium IV nur noch in Form einer intraarteriellen Infusion gegeben werden.

Bei dem in der oben genannten Studie verwendeten Produkt Prostavasin® handelt es sich um ein Lyophilisat, also um ein festes, pulverförmiges Konzentrat, das durch Gefriertrocknung des Wirkstoffes zusammen mit stabilisierenden Hilfsstoffen hergestellt wurde. Als stabilisierender Hilfsstoff wird bei Prostavasin® (UCB Pharma) alpha-Cyclodextrin (Alfadex) verwendet. Weitere Handelsbezeichnungen solcher alpha-cyclodextrinhaltiger Produkte sind z.B. Vasoprost® und Sugiran®. Auch Alprostadil Injektionspräparate zur Behandlung der erektilen Dysfunktion werden in dieser Technologie hergestellt, z.B. Caverject® (Pfizer, Pharmacia) oder Viridal® (UCB Pharma). Die CN 102688203 beschreibt ein Lyophilisat enthaltend Alprostadil, bei dem zur Stabilisierung eine Mischung von Dextran und Hydroxypropyl-beta-Cyclodextrin verwendet wird.

Cyclodextrine sind im Europäischen Arzneibuch monographierte Hilfsstoffe, bei deren Verwendung in Arzneimitteln derzeit kein besonderer Hinweis im Beipackzettel des Arzneimittels vorgeschrieben ist. Dennoch gibt es gegenüber diesem Hilfsstoff Sicherheitsbedenken, die dazu geführt haben, dass derzeit innerhalb der EMA (European Medicines Agency) diskutiert wird, bei Verwendung von Cyclodextrinen in Arzneimitteln einen Warnhinweis im Beipackzettel verpflichtend vorzuschreiben.

Die Bewertung des toxikologischen Profils der Cyclodextrine hat differenziert nach den verschiedenen Applikationsarten und den verschiedene Typen der Cyclodextrine zu erfolgen. Bei der parenteralen Applikation erfolgt eine relativ rasche Elimination über die Niere mit einer Halbwertszeit von 20 - 100 Minuten. Dabei ist die Nephrotoxizität der Substanzen von spezieller Relevanz. Während die Nephrotoxizität von Hydroxypropyl-ß-cyclodextrin und von Sulfobutylether-β-cyclodextrin relativ gering zu sein scheint, sind das im Prostavasin® verwendete α-Cyclodextrin und insbesondere das β-Cyclodextrin bereits in geringen Konzentrationen nephrotoxisch. In ihrem Positionspapier "Background review for cyclodextrines used as excipients" vom 20. November 2014 kommt die EMA deshalb zu folgendem Schluss "Sowohl α- als auch β-Cyclodextrin zeigen eine Nierentoxizität bei parenteraler Verabreichung und sind deshalb in der Regel nicht für Arzneimittel geeignet, die intravenös gegeben werden". Dieses Urteil gilt in gleicher Weise für die intraarterielle Anwendung, da die Elimination auf die gleiche Weise erfolgt.

In Zukunft ist deshalb damit zu rechnen, dass der Einsatz von Cyclodextrinen in Arzneimittel, insbesondere in parenteral anzuwenden Arzneimitteln, bei der Zulassung einer strengeren toxikologischen Bewertung unterzogen wird. Die Einführung eines verpflichtenden Warnhinweises im Beipackzettel für alle cyclodextrinhaltigen Arzneimittel ist sehr wahrscheinlich. Eine untere, unkritische Expositionsgrenze ist derzeit nicht bekannt und wird vermutlich nicht definiert werden; aus toxikologischer Sicht ist vielmehr die vollständige Vermeidung der Substanz (zero threshold) das Ziel.

Bei der Behandlung von Patienten mit peripherer arterieller Verschlusskrankheit ist diese allgemeine Problematik der Nephrotoxizität von α-Cyclodextrin dadurch verschärft, dass viele dieser Patienten bereits eine vorgeschädigte Niere und dadurch eine zusätzliche Empfindlichkeit gegen weitere Noxen aufweisen. Zum einen können die gleichen Faktoren, die zur Ausbildung der peripheren arteriellen Verschlusskrankheit führen, auch zu einer renalen arteriellen Verschlusskrankheit führen, zum anderen gilt eine bestehende Niereninsuffizienz als Risikofaktor für die pAVK. Ganz allgemein ist darüber hinaus festzustellen, dass es eine Reihe von Risikofaktoren gibt, die sowohl die periphere arterielle Verschlusskrankheit als auch Funktionseinschränkungen der Niere begünstigen, sodass die Koinzidenz beider Krankheitsbilder nicht unwahrscheinlich ist.

Aus den vorstehenden Ausführungen ergibt sich, dass es insbesondere für die Behandlung der Stadien III und IV der pAVK einen großen Bedarf für eine parenteral anwendbare Alprostadil Zubereitung gibt, die intraarteriell anwendbar ist und die frei von Cyclodextrinen ist. Dieser Bedarf wird durch die oben beschriebenen und durch die bisher im Markt verfügbaren Produkte aus den angeführten Gründen nicht befriedigt.

Auf Basis dieser Überlegungen und der im Stand der Technik dargestellten Vorurteile, Bedenken und Probleme sowohl bei der Verwendung von alkoholischen Alprostadil-Konzentraten, u.a. wegen vermeintlicher lokaler Unverträglichkeiten, als auch bei der Verwendung von cyclodextrinhaltigen Alprostadil-Lyophilisaten, u.a. wegen der neu erkannten renalen Toxizität, hat die Anmelderin das Risiko-Nutzen-Verhältnis von alkoholischen Alprostadil-Zubereitungen und insbesondere deren intraarteriellen Verwendung einer neuen Bewertung unterzogen.

Aufgabe der vorliegenden Erfindung war es somit, eine Zusammensetzung und deren Verwendung zur Behandlung der peripheren arteriellen Verschlusskrankheit zu schaffen, die eine gute lokale Verträglichkeit aufweist und bei der eine hohe Wirkstoffkonzentration speziell und direkt am Wirkort, nämlich v.a. den Arterien der unteren Extremitäten, gewährleistet wird.

Bei den nachfolgend dargestellten Untersuchungen wurde insbesondere die lokale Verträglichkeit der ethanolischen Alprostadil Lösung, bzw. der aus dem ethanolischen Alprostadil Konzentrat durch Verdünnung hergestellten, alkoholhaltigen Infusionslösung, an den Gefäßen trotz der bestehenden negativen Vorurteile experimentell untersucht.

Die lokale Verträglichkeit der ausgehend von den Pridax®-Ampullen (ethanolisches Konzentrat) hergestellten Infusionslösung wurde im Vergleich zur lokalen Verträglichkeit der ausgehend von Prostavasin® (mit α-Cyclodextrin stabilisiertes Konzentrat) hergestellten rein wässrigen Infusionslösung getestet. Die vergleichende Prüfung erfolgte tierexperimentell entsprechend der EMA Richtlinie CPMP/SWP/2145/00 (Note für Guidance on Non-Clinical Local Tolerance Testing of Medicinial Products). Beschrieben wird in dieser Richtlinie die Prüfung der lokalen Verträglichkeit von Arzneimitteln an dem Ort des Körpers, der mit dem Arzneimittel im Zuge der klinischen Anwendung in Kontakt kommt. Für die Verträglichkeitsprüfung bei parenteraler, intraarterieller Applikation schlägt die Richtlinie die Prüfung an der Zentralarterie des Kaninchenohrs als Testmodell vor.

Die Studie wurde über 3 respektive über 8 Tage an jeweils 3 weißen New Zealand Kaninchen mit der Pridax® Medikation und der Prostavasin® Medikation durchgeführt (insgesamt 12 Tiere). Es wurden jeweils 1 Ampulle Pridax® oder Prostavasin®, enthaltend jeweils 20 µg Alprostadil, in 50 ml physiologischer Kochsalzlösung gelöst. Diese Lösungen mit einer Wirkstoffkonzentration von jeweils 0,4 µg/ml wurden jeweils über 2 Stunden infundiert (0,417 ml/min). Als Ergebnisbefunde wurden klinische Beobachtungen herangezogen, die makroskopische Untersuchung der Infusionsstelle und eine histologische Untersuchung der Infusionsstelle und der wichtigsten inneren Organe.

Keines der Versuchstiere zeigte klinische Erkrankungs- oder Vergiftungssymptome; keines der Tiere starb.

Drei Tag nach der Infusion zeigten die Tiere eine lokale Irritation an der Einstichstelle und in einem gewissen Bereich entlang der Gefäße in Form einer Rötung des Epithelgewebes. Ein signifikanter Unterschied zwischen der Pridax® Gruppe und der Prostavasin® Gruppe wurde nicht beobachtet. Acht Tage nach der Infusion waren die Irritationen bei allen Tieren weitgehend abgeklungen.

Bei der histologischen Untersuchung der Arterienwand zeigten sich bei den meisten Tieren Hautirritationen an der Einstichstelle und arterielle Thrombosen. Die Bildung dieser Thrombosen war bei der Pridax® Gruppe signifikant geringer ausgeprägt als bei der Prostavasin® Gruppe.

Entgegen der Erwartung wurde also bei diesen Untersuchungen gefunden, dass zwar die intraarterielle Infusion beider Zubereitungen zu lokalen Irritationen der Arterienwand und der Bildung von arteriellen Thrombosen führt, dass dieser Effekt aber bei der Gabe der alkoholischen Pridax® Lösung geringer ausgeprägt war als bei der Gabe der α-cyclodextrinhaltigen Prostavasin® Lösung. Die lokale Verträglichkeit von Pridax® war also nicht nur genauso gut wie die von Prostavasin®, sondern überraschenderweise sogar besser.

Überraschend hat sich gezeigt, dass eine flüssige pharmazeutische Zusammensetzung in Form eines Konzentrats zur Herstellung einer Infusionslösung, enthaltend Alprostadil (Prostaglandin E1) als Wirkstoff, gelöst in einem pharmazeutisch annehmbaren organischen Lösungsmittel oder in einem Lösungsmittelgemisch aus zumindest einem pharmazeutisch annehmbaren organischen Lösungsmittel und Wasser, zur Verwendung in der Behandlung der peripheren arteriellen Verschlusskrankheit (pAVK) mittels intraarterieller (i.a.) Infusion vorteilhaft anwendbar ist und dass die oben gestellte Aufgabe auf diese Weise lösbar ist. Die Erfindung betrifft also eine derartige flüssige pharmazeutische Zusammensetzung zur Verwendung in der Behandlung bzw. zur Anwendung in einem Verfahren für die Behandlung oder bei der Therapie der peripheren arteriellen Verschlusskrankheit (pAVK) mittels intraarterieller (i.a.) Infusion.

Bei der intraarteriellen (i.a.) Infusion gelangt der Wirkstoff rasch und unverdünnt in die arteriellen Endgefäße und damit an seinen Wirkort. Die i.a. Applikation stellt somit für das Alprostadil eine zielgerichtete Applikationsform dar, während die i.v. Applikation einer unspezifischen systemischen Applikation gleich kommt. Die durch die i.a. Verabreichung erzielbaren höheren lokalen Plasmaspiegel gehen auch mit einer stärkeren Wirkung, d.h. einer ausgeprägteren Gefäßdilatation einher, was den mit dieser Applikationsform verbundenen höheren klinischen Aufwand und die höhere Belastung der Patienten rechtfertigt.

Die erfindungsgemäß aus einem rein alkoholischen bzw. einem alkoholisch/wässrigen Konzentrat durch Verdünnung mit physiologischer Kochsalzlösung hergestellte gebrauchsfertige Infusionslösung eignet sich vorteilhafterweise zur Behandlung aller pAVK Krankheitszustände, bei denen die intraarterielle Gabe von Alprostadil indiziert ist, also zur Behandlung der Stadien IIb, III und insbesondere IV der periphere arterielle Verschlusskrankheit.

Entsprechend der bei der Therapie der pAVK angestrebten Zielregion erfolgt die intraarterielle Infusion der Lösung vorteilhafterweise in die Leistenarterie, kann aber auch über die Beckenarterie(n), Unterschenkelarterie(n), Oberschenkelarterie(n) oder Kniearterie(n) erfolgen. Dabei können Einmalkatheter oder Dauerkatheter zum Einsatz kommen. Die Infusionsdauer beträgt normalerweise bei Einsatz einer Spritzenpumpe (z.B. Perfusor) mindestens 1 h, die Infusionsdauer kann aber bei Verwendung einer Dauerinfusionspumpe auch auf 12 Stunden oder mehr ausgedehnt werden.

Die Dosierung bei der intraarteriellen Gabe von Alprostadil beträgt vorteilhafterweise etwa 10 µg Alprostadil / Tag. Je nach Schwere der Erkrankung, Konstitution des Patienten und individueller Verträglichkeit kann die optimale Gesamtdosis vorteilhafterweise aber zwischen 5 und 40 µg, insbesondere zwischen 10 und 20 µg, Alprostadil / Tag schwanken, wobei die Verabreichung vorteilhafterweise einmal täglich erfolgt. Dosierungen über 20 µg Alprostadil / Tag werden insbesondere in Fällen gegeben, in denen die Infusion mittels einer Infusionspumpe über einen längeren Zeitraum von z.B. 12 Stunden erfolgt. Noch höhere oder noch niedrigere Dosierungen sind nicht ausgeschlossen, bleiben aber in der therapeutischen Praxis Einzelfällen vorbehalten. Aufgrund der höheren therapeutischen Verfügbarkeit sind die intraarteriell gegebenen Dosierungen jedenfalls kleiner als die üblicherweise intravenös gegebenen.

Vorteilhaft ist eine Zufuhrrate, entsprechend maximal 0,8 g organisches Lösungsmittel, insbesondere Ethanol, pro Stunde.

Vorteilhafterweise ist vorgesehen, dass der Anteil des organischen Lösungsmittels im Lösungsmittelgemisch zumindest 1 Gew.% bezogen auf die Gesamtmenge des Lösungsmittelgemisches, ist. Auch höhere Anteile an organischen Lösungsmitteln im Lösungsmittelgemisch sind möglich, beispielsweise von zumindest 40 Gew.% oder zumindest 80 Gew.% bezogen auf die Gesamtmenge des Lösungsmittelgemisches. Durch entsprechende Anteile an organischen Lösungsmitteln werden eine bessere Löslichkeit des Wirkstoffs gewährleistet und die Stabilität erhöht. Insbesondere vorgesehen ist deshalb auch die Verwendung eines rein organischen Lösungsmittels, wie sie bei den derzeit verfügbaren Konzentraten, z.B. Pridax®, realisiert ist
Der Begriff "pharmazeutisch annehmbares organisches Lösungsmittel" umfasst mit Wasser in den verwendeten Konzentrationsbereichen mischbare, für den Menschen physiologisch verträgliche und hinsichtlich ihrer Qualität pharmazeutisch akzeptable organische Lösungsmittel. Unter dem Begriff "organische Lösungsmittel" werden dabei auch solche organische Lösungsmittel subsumiert, die Wasser wegen ihrer Hygroskopizität als Verunreinigung enthalten, z.B. absoluter Ethanol, oder die inhärent einen gewissen Anteil an Wasser enthalten, z.B. der häufig verwendete, azeotrope 96 %ige (V/V) Ethanol.

Bei den Angaben des Gesamt-Wasseranteils bzw. des Wassergehalts in Gew.% handelt es sich vorliegend stets um den effektiven Wassergehalt, also den tatsächlichen Wasseranteil im Lösungsmittelgemisch. Allenfalls inhärent im organischen Lösungsmittel bereits enthaltenes Wasser im obigen Sinn ist dabei berücksichtigt und von den Angaben in Gew.% umfasst.

In diesem Zusammenhang ist es möglich, dass der Gesamt-Wasseranteil im Lösungsmittelgemisch, insbesondere in einem Gemisch mit Ethanol abs. oder Ethanol 96% als Lösungsmittel, im Bereich von 10 bis 60 Gew.% H₂O, vorzugsweise von 20 bis 60 Gew.% H₂O, insbesondere von 45 bis 55 Gew.% H₂O, vorzugsweise bei 50 Gew.% H₂O, bezogen auf die Gesamtmenge des Lösungsmittelgemisches, liegt. Bei diesen Mischungsverhältnissen, vor allem im Bereich um etwa 50 Gew% H₂O, ist die Stabilität des Konzentrats dem derzeitigen rein ethanolischen Stand der Technik sogar überlegen und die zu erwartenden medizinischen Vorteile einer Reduktion des organischen Lösungsmittels im Bereich der lokalen Verträglichkeit sind bereits signifikant. Eine solche Zusammensetzung mit einem geringeren Alkoholanteil besitzt zudem wegen der verbesserten Stabilität eine längere Haltbarkeit und enthält eine geringere Konzentration an Abbauprodukten.

In diesem Zusammenhang ist vorteilhaft vorgesehen, dass das, insbesondere jedes, organische Lösungsmittel, in den verwendeten Konzentrationsbereichen vollständig mit Wasser mischbar ist und/oder dass das Lösungsmittelgemisch aus dem zumindest einen organischen Lösungsmittel und Wasser in den verwendeten Konzentrationsbereichen eine echte einphasige Lösung ist.

Vorteilhafterweise und besonders bevorzugt sind als organische Lösungsmittel Ethanol 96 % PhEur (95.1 - 96.9 % (V/V) entsprechend 92,6 - 95,2 Gew.-%) oder Wasserfreies Ethanol PhEur (min. 99.5 % (V/V) entsprechend min. 99,2 Gew.-%) bzw. absoluter Ethanol einsetzbar, möglich ist auch die alleinige oder anteilsweise Verwendung von organischen Lösungsmitteln aus der Gruppe Propylenglycol, 1,3 Butandiol, Ethylacetat und Dimethylisosorbid, soweit zutreffend jeweils gemäß den Definitionen und Anforderungen der Europäischen Pharmakopöe. All diese Lösungsmittel sind in parenteralen Zubereitungen auf Grund ihres toxikologischen Profils anwendbar und der Wirkstoff Alprostadil ist darin in ausreichender Konzentration löslich. Auch Mischungen dieser Lösungsmittel sind vorteilhaft einsetzbar. Darüber hinaus können dem Lösungsmittel auch Lösungsvermittler wie Polyethylenglycol oder Polysorbat zugesetzt sein.

Eine besonders vorteilhafte Ausgestaltung sieht eine flüssige pharmazeutische Zusammensetzung in Form eines Konzentrats zur Herstellung einer Infusionslösung vor, bestehend abschließend und ausschließlich aus Alprostadil (Prostaglandin E1) als Wirkstoff, gelöst in einem pharmazeutisch annehmbaren organischen Lösungsmittel oder in einem Lösungsmittelgemisch aus zumindest einem pharmazeutisch annehmbaren organischen Lösungsmittel und Wasser.
Vor allem ist es vorteilhaft, wenn keine weiteren Bestandteile, Exzipienten oder Additive umfasst sind bzw. wenn die Zusammensetzung frei von weiteren Bestandteilen, Exzipienten oder Additiven ist. Insbesondere ist es vorteilhaft, wenn sie frei von Salzen und/oder Isotonisierungszusätzen ist.

Ganz besonders vorteilhaft ist es, wenn die Zusammensetzung frei von Cyclodextrinen ist, insbesondere von α-Cyclodextrin und β-Cyclodextrin. Überraschenderweise wurde nämlich, wie einleitend beschrieben, gefunden, dass eine besonders vorteilhafte Zusammensetzung, die die nach dem bisherigen Stand der Technik unvermeidliche toxikologische Belastung durch Hilfsstoffe aus der Gruppe der Cyclodextrine bewusst vermeidet, im Vergleich zum Stand der Technik, insbesondere im Vergleich zu einer Lösung hergestellt aus dem Lyophilisat Prostavasin® - Trockensubstanz zur Infusionsbereitung, enthaltend Alprostadil in Form einer α-Cyclodextrin Einschlussverbindung, nach intraarterieller Applikation eine bessere lokale Verträglichkeit, insbesondere eine geringere Neigung intraarterielle Thrombosen zu verursachen, aufweist. Die beobachtete Verbesserung der Verträglichkeit ist signifikant. Der Fortschritt gegenüber dem Stand der Technik besteht dabei einerseits in der besseren lokalen Verträglichkeit und andererseits in der Vermeidung der toxikologischen, insbesondere der nephrotoxischen Risiken, die mit der parenteralen Gabe von α-Cyclodextrin als Hilfsstoff verbunden sind.

Ein vorteilhaftes Konzentrat liegt vor, wenn Alprostadil in einer Konzentration von 1-1000 µg/ml, vorzugsweise 5-750 µg/ml, insbesondere 20-500 µg/ml enthalten ist.

Dem Stand der Technik entsprechend wird die Konzentration des Wirkstoffes in Alprostadil-Lösungen in der Einheit µg/ml angegeben. Eine Umrechnung in die Einheit µg/g kann jederzeit vorgenommen werden, indem durch die Dichte des Lösungsmittelgemisches dividiert wird.

Die Abfüllung des erfindungsgemäßen Konzentrats erfolgt in geeignete Primärpackmittel. Als Primärpackmittel werden in diesem Zusammenhang Packmittel verstanden, welche in direktem Kontakt mit dem Arzneimittel stehen. Sie müssen einerseits den Anforderungen der einschlägigen Arzneibücher entsprechen und müssen darüber hinaus so ausgewählt werden, dass sie möglichst wenig für die Stabilität des Inhalts schädliche Substanzen, wie z.B. basische Verunreinigungen, abgeben. Geeignete Materialien, aus denen für empfindliche Parenteralia einsetzbare Primärpackmittel bestehen, sind dem Fachmann bekannt. Denkbar sind beispielsweise, aber nicht ausschließlich Röhrenglas oder Hüttenglas der hydrolytischen Klasse I oder der hydrolytischen Klasse II, mit Innenvergütung. Ebenso anwendbar sind beispielsweise, aber nicht ausschließlich Kunststoffe wie COP, COC, PP, PE, HDPE, Teflon, PA.

Bevorzugte Primärbehältnisse sind Glasampullen oder Durchstechflaschen (Vials), bestehend aus Röhrenglas der hydrolytischen Klasse I. Als primäres Verschlussmaterial für Durchstechflaschen kommen Stopfen aus Brombutyl-Kautschuk oder Chlorbutyl-Kautschuk, vorzugsweise beschichtet, in Frage. Weitere mögliche Primärbehältnisse sind beispielsweise, aber nicht ausschließlich, Karpulen oder Fertigspritzen.

Daraus ergibt sich eine vorteilhafte Kit-of-parts-Kombination, wenn die erfindungsgemäße Zusammensetzung abgefüllt bzw. enthalten in einer dicht verschlossenen Glasampulle, Glasvial, Karpule oder Fertigspritze, vorliegt.

Die erfindungsgemäße pharmazeutische Zusammensetzung ist vorteilhafterweise ein Konzentrat für die Erstellung einer parenteral zu verabreichenden Infusionslösung, welches insbesondere durch einen Verdünnungsschritt, zu der gebrauchsfertigen Infusionslösung für die parenterale Verabreichung herrichtbar oder hergerichtet ist. Durch die Verdünnung, z.B. mit gegebenenfalls isotonisierter, wässriger Lösung, vorzugsweise mit physiologischer Kochsalzlösung, insbesondere in einem Verhältnis von 1:10 bis 1:250, vorzugsweise 1:50, ist dann die gebrauchsfertige parenterale Infusionslösung erhältlich.
Die vorstehend angegebenen Konzentrationsangaben, z.B. für Alprostadil etc. beziehen sind auf ein solches Konzentrat und nicht auf die gebrauchsfertige, verdünnte Infusionslösung.

Erfindungsgemäß ist weiters auch eine gebrauchsfertige Infusionslösung vorgesehen, erhältlich durch Verdünnen der erfindungsgemäßen Zusammensetzung mit einer geeigneten Trägerlösung, insbesondere mit isotonisierter, wässriger Lösung, vorzugsweise mit physiologischer Kochsalzlösung, wässriger Glucoselösung oder isotonen Elektrolyt-Infusionslösungen, beispielsweise Ringerlösung oder Ringer-Lactat-Lösung, insbesondere in einem Verhältnis von 1:10 bis 1:250, vorzugsweise 1:50, zur Verwendung in der Behandlung der peripheren arteriellen Verschlusskrankheit (pAVK) mittels intraarterieller (i.a.) Infusion. Gleichermaßen ist erfindungsgemäß auch die Verwendung einer solchen gebrauchsfertigen Infusionslösung zur Herstellung eines Medikamentes zur Behandlung der peripheren arteriellen Verschlusskrankheit (pAVK) mittels intraarterieller (i.a.) Infusion vorgesehen.

Dabei ist vorteilhafterweise vorgesehen, dass in der gebrauchsfertigen Infusionslösung Alprostadil in einer Konzentration von 0,04 bis 2 µg/ml, vorzugsweise 0,05 bis 1 µg/ml, insbesondere 0,08 bis 0,8 µg/ml, enthalten ist. Die Verdünnung des Alprostadil Konzentrats erfolgt besonders bevorzugt so, dass sich in der Infusionslösung eine Alprostadil Konzentration von 0,4 µg/ml einstellt. In speziellen Fällen kann diese Konzentration bis auf 0,8 µg/ml erhöht oder bis auf 0,08 µg/ml reduziert werden. Höhere oder noch niedrigere Konzentrationen sind nicht ausgeschlossen, bleiben aber in der therapeutischen Praxis Einzelfällen vorbehalten.

Besonders vorteilhaft ist es, wenn 1 ml der erfindungsgemäßen Zusammensetzung in einer Verdünnung mit 50 ml isotonischer Natriumchloridlösung verwendet wird.

Vorteilhaft ist eine Zufuhrrate bzw. Verabreichungsgeschwindigkeit, entsprechend maximal 0,8 g organisches Lösungsmittel, insbesondere Ethanol, pro Stunde.

Die Infusionsgeschwindigkeit ist von vielerlei Faktoren abhängig, insbesondere von der zu verabreichenden Dosis, der Schwere der Erkrankung, der Konstitution des Patienten und der individueller Verträglichkeit und der Art des verwendeten intraarteriellen Katheters sowie der Art der Zuführung der Infusionslösung. Vorteilhafterweise ist vorgesehen, dass die Verabreichung mit einer Zufuhrrate von 20 - 500 ng Alprostadil pro Minute und/oder mit einer Infusionsgeschwindigkeit von 0,1 bis 1 ml Infusionslösung/min erfolgt.

Bei einer beispielhaften Standardtherapie werden 10 µg Alprostadil in 25 ml physiologischer Kochsalzlösung verdünnt und mittels Perfusor über 60 -120 min i.a. infundiert. Daraus ergibt sich bei einer Infusionsdauer von 60 min eine Infusionsgeschwindigkeit von etwa 160 ng/min, bei einer Infusionsdauer von 120 min eine Infusionsgeschwindigkeit von etwa 80 ng/min.

Bei Bedarf und guter Verträglichkeit kann die Alprostadil Dosierung auf 20 µg erhöht werden. In diesem Fall wird der Wirkstoff vorzugsweise in 50 ml physiologischer Kochsalzlösung gelöst und die Infusionsdauer wird bei 60 - 120 min belassen. Daraus ergeben sich doppelt so hohe Infusionsgeschwindigkeiten von ca. 320 bzw. 160 ng/min.

Eine weitere Möglichkeit besteht darin, die Medikation über einen Verweilkatheter mittels Infusionspumpe zu infundieren. In diesem Fall wird die Infusionsdauer auf z.B. 12 h ausgedehnt und die resultierende Infusionsgeschwindigkeit liegt ja nach Dosierung und Patient bei 0,1 - 0,6 ng/kgKG/min.

### Beispiele:

### Beispiel 1:

Eine Ampulle eines ethanolischen Alprostadil Konzentrats, enthaltend 20 µg Alprostadil gelöst in 1 ml wasserfreiem Ethanol werden mit 50 ml physiologischer Kochsalzlösung verdünnt. Von dieser wirkstoffhaltigen Infusionslösung mit einer Konzentration ca. 0,4 µg/ml werden 25,5 ml innerhalb von 60 Minuten mittels eines Perfusors in die Leistenarterie infundiert. Dies entspricht einer Einmaldosis von 10 µg Alprostadil, die mit einer Infusionsgeschwindigkeit von ca. 0,425 ml/min bzw. von ca. 167 ng/min verabreicht werden.

### Beispiel 2:

Eine Ampulle eines ethanolischen Alprostadil Konzentrats, enthaltend 20 µg Alprostadil gelöst in 1 ml wasserfreiem Ethanol werden mit 50 ml physiologischer Kochsalzlösung verdünnt. Von dieser wirkstoffhaltigen Infusionslösung mit einer Konzentration ca. 0,4 µg/ml werden 38,2 ml innerhalb von 90 Minuten mittels eines Perfusors in die Leistenarterie infundiert. Dies entspricht einer Einmaldosis von 15 µg Alprostadil, die mit einer Infusionsgeschwindigkeit von ca. 0,425 ml/min bzw. von ca. 167 ng/min verabreicht werden.

### Beispiel 3:

Eine Ampulle eines ethanolischen Alprostadil Konzentrats, enthaltend 20 µg Alprostadil gelöst in 1 ml wasserfreiem Ethanol werden mit 50 ml Ringer-Lactat Infusionslösung verdünnt. Diese wirkstoffhaltige Infusionslösung mit einer Konzentration ca. 0,4 µg/ml wird innerhalb von 120 Minuten mittels eines Perfusors in die Leistenarterie infundiert. Dies entspricht einer Einmaldosis von 20 µg Alprostadil, die mit einer Infusionsgeschwindigkeit von ca. 0,425 ml/min bzw. von ca. 167 ng/min verabreicht werden.

### Beispiel 4:

Eine Ampulle eines ethanolischen/wässrigen Alprostadil Konzentrats, enthaltend 20 µg Alprostadil gelöst in 1 ml einer Mischung aus 50 % Gew. wasserfreiem Ethanol und 50 % Gew. Wasser werden mit 50 ml physiologischer Kochsalzlösung verdünnt. Von dieser wirkstoffhaltigen Infusionslösung mit einer Konzentration ca. 0,4 µg/ml werden 25,5 ml innerhalb von 120 Minuten mittels eines Perfusors in die Leistenarterie infundiert. Dies entspricht einer Einmaldosis von 10 µg Alprostadil, die mit einer Infusionsgeschwindigkeit von ca. 0,21 ml/min bzw. von ca. 83 ng/min verabreicht werden.

### Beispiel 5:

Eine Ampulle eines ethanolischen Alprostadil Konzentrats, enthaltend 20 µg Alprostadil gelöst in 1 ml Ethanol 96 % (V/V) werden mit 50 ml physiologischer Kochsalzlösung verdünnt. Diese wirkstoffhaltige Infusionslösung mit einer Konzentration ca. 0,4 µg/ml wird innerhalb von 60 Minuten mittels eines Perfusors in die Leistenarterie infundiert. Dies entspricht einer Einmaldosis von 20 µg Alprostadil, die mit einer Infusionsgeschwindigkeit von ca. 0,85 ml/min bzw. von ca. 333 ng/min verabreicht werden.

### Beispiel 6:

Eine halbe Ampulle eines ethanolischen Alprostadil Konzentrats, enthaltend 10 µg Alprostadil gelöst in 0,5 ml wasserfreiem Ethanol werden mit 50 ml physiologischer Kochsalzlösung verdünnt. Diese wirkstoffhaltige Infusionslösung mit einer Konzentration ca. 0,2 µg/ml wird innerhalb von 120 Minuten mittels eines Perfusors in die Leistenarterie infundiert. Dies entspricht einer Einmaldosis von 10 µg Alprostadil, die mit einer Infusionsgeschwindigkeit von ca. 0,42 ml/min bzw. von ca. 83 ng/min verabreicht werden.

### Beispiel 7:

Zwei Ampullen eines ethanolischen Alprostadil Konzentrats, enthaltend 40 µg Alprostadil gelöst in 2 ml wasserfreiem Ethanol werden mit 50 ml physiologischer Kochsalzlösung verdünnt. Diese wirkstoffhaltige Infusionslösung mit einer Konzentration ca. 0,8 µg/ml wird innerhalb von 120 Minuten mittels eines Perfusors in die Leistenarterie infundiert. Dies entspricht einer Einmaldosis von 40 µg Alprostadil, die mit einer Infusionsgeschwindigkeit von ca. 0,433 ml/min bzw. von ca. 333 ng/min verabreicht werden.

### Beispiel 8:

Eine Ampulle eines ethanolischen Alprostadil Konzentrats, enthaltend 20 µg Alprostadil gelöst in 1 ml wasserfreiem Ethanol werden mit 250 ml physiologischer Kochsalzlösung verdünnt. Von dieser wirkstoffhaltigen Infusionslösung mit einer Konzentration ca. 0,08 µg/ml werden 125,5 ml innerhalb von 360 Minuten mittels einer Infusionspumpe in die Leistenarterie infundiert. Dies entspricht einer Dosis von 10 µg Alprostadil, die mit einer Infusionsgeschwindigkeit von ca. 0,35 ml/min bzw. von ca. 27,8 ng/min verabreicht werden.

### Beispiel 9:

Zwei Ampulle eines ethanolischen Alprostadil Konzentrats, enthaltend 40 µg Alprostadil gelöst in 2 ml wasserfreiem Ethanol werden mit 100 ml physiologischer Kochsalzlösung verdünnt. Diese wirkstoffhaltige Infusionslösung mit einer Konzentration ca. 0,4 µg/ml wird innerhalb von 12 Stunden mittels einer Infusionspumpe in die Leistenarterie infundiert. Dies entspricht einer Dosis von 40 µg Alprostadil, die mit einer Infusionsgeschwindigkeit von ca. 0,142 ml/min bzw. von ca. 55 ng/min verabreicht werden.

### Beispiel 10:

Eine Ampulle eines ethanolischen Alprostadil Konzentrats, enthaltend 20 µg Alprostadil gelöst in 1 ml wasserfreiem Ethanol werden mit 50 ml physiologischer Kochsalzlösung verdünnt. Von dieser wirkstoffhaltigen Infusionslösung mit einer Konzentration ca. 0,4 µg/ml werden 12,75 ml innerhalb von 60 Minuten mittels eines Perfusors in die Leistenarterie infundiert. Dies entspricht einer Einmaldosis von 5 µg Alprostadil, die mit einer Infusionsgeschwindigkeit von ca. 0,21 ml/min bzw. von ca. 83 ng/min verabreicht werden.

## Patentansprüche

1. Flüssige pharmazeutische Zusammensetzung in Form eines Konzentrats zur Herstellung einer Infusionslösung, enthaltend, insbesondere bestehend aus, Alprostadil (Prostaglandin E1) als Wirkstoff, gelöst in einem pharmazeutisch annehmbaren organischen Lösungsmittel oder in einem Lösungsmittelgemisch aus zumindest einem pharmazeutisch annehmbaren organischen Lösungsmittel und Wasser, zur Verwendung in der Behandlung der peripheren arteriellen Verschlusskrankheit (pAVK) mittels intraarterieller (i.a.) Infusion.

2. Zusammensetzung zur Anwendung nach Anspruch 1 zur Verwendung in der Behandlung der peripheren arteriellen Verschlusskrankheit (pAVK) im Stadium IIb, III und/oder IV gemäß der Klassifikation von Fontaine.

3. Zusammensetzung zur Anwendung nach einem der Ansprüche 1 oder 2, zur Verwendung für eine Verabreichung in die Beckenarterie(n), Unterschenkelarterie(n), Oberschenkelarterie(n), Kniearterie(n) und/oder Leistenarterie(n).

4. Zusammensetzung zur Anwendung nach einem der Ansprüche 1 bis 3, zur Verwendung einmal täglich, in einer Gesamtdosis von 5 bis 40 µg, insbesondere 10 bis 20 µg, Alprostadil pro Tag,
und /oder
zur Verwendung mit einer Zufuhrrate, entsprechend maximal 0,8 g organisches Lösungsmittel, insbesondere Ethanol, pro Stunde.

5. Zusammensetzung zur Anwendung nach einem der Ansprüche 1 bis 4, **dadurch gekennzeichnet, dass** der Anteil des organischen Lösungsmittels im Lösungsmittelgemisch zumindest 1 Gew.% bezogen auf die Gesamtmenge des Lösungsmittelgemisches, ist und/oder dass der Anteil des organischen Lösungsmittels im Lösungsmittelgemisch zumindest 40 Gew.% oder zumindest 80 Gew.% bezogen auf die Gesamtmenge des Lösungsmittelgemisches, ist.

6. Zusammensetzung zur Anwendung nach einem der Ansprüche 1 bis 5, **dadurch gekennzeichnet, dass** der Gesamt-Wasseranteil im Lösungsmittelgemisch, insbesondere in einem Gemisch mit Wasserfreiem Ethanol und/oder Ethanol 96% als Lösungsmittel, im Bereich von 10 bis 60 Gew.% H₂O, vorzugsweise von 20 bis 60 Gew.% H₂O, insbesondere von 45 bis 55 Gew.% H₂O, vorzugsweise bei 50 Gew.% H₂O, bezogen auf die Gesamtmenge des Lösungsmittelgemisches, liegt.

7. Zusammensetzung zur Anwendung nach einem der Ansprüche 1 bis 6, **dadurch gekennzeichnet, dass** das, insbesondere jedes, organische Lösungsmittel, insbesondere vollständig, mit Wasser mischbar ist bzw. dass das Lösungsmittelgemisch aus dem zumindest einen organischen Lösungsmittel und Wasser eine echte einphasige Lösung ist.

8. Zusammensetzung zur Anwendung nach einem der Ansprüche 1 bis 7, **dadurch gekennzeichnet, dass** das organische Lösungsmittel ausgewählt ist aus der Gruppe von Propylenglycol, 1,3 Butandiol, Ethylacetat, Dimethylisosorbid, Ethanol 96 % PhEur (95.1 - 96.9 % (V/V)) oder Wasserfreies Ethanol PhEur (min. 99.5 % (V/V)) bzw. absoluter Ethanol oder Mischungen davon, wobei als organisches Lösungsmittel Ethanol 96 % PhEur (95.1 - 96.9 % (V/V)) und/oder Wasserfreies Ethanol PhEur (min. 99.5 % (V/V)) bzw. absoluter Ethanol besonders bevorzugt sind.

9. Zusammensetzung zur Anwendung nach einem der Ansprüche 1 bis 8, **dadurch gekennzeichnet, dass** die Zusammensetzung frei von Cyclodextrinen, insbesondere frei von α-Cyclodextrin und/oder β-Cyclodextrin, ist
und/oder
dass die Zusammensetzung frei von weiteren Bestandteilen, Exzipienten oder Additiven, insbesondere frei von Salzen und/oder Isotonisierungszusätzen ist.,
und/oder
dass Alprostadil in einer Konzentration von 1-1000 µg/ml, vorzugsweise 5-750 µg/ml, insbesondere 20-500 µg/ml enthalten ist.

10. Zusammensetzung zur Anwendung nach einem der Ansprüche 1 bis 9, **dadurch gekennzeichnet, dass** diese ein Konzentrat zur Herstellung einer gebrauchsfertigen Infusionslösung für die parenterale Verabreichung, insbesondere durch einen Verdünnungsschritt mit einer geeigneten Trägerlösung, ist oder als Konzentrat zur weiteren Herstellung einer verdünnten, gebrauchsfertigen Infusionslösung für die parenterale Verabreichung hergerichtet ist und/oder
dass diese in Glasampullen, Glasvials, Karpulen oder Fertigspritzen abgefüllt ist.

11. Kit-of-parts, enthaltend die Zusammensetzung zur Verwendung nach einem der Ansprüche 1 bis 10, enthalten in einer verschlossenen Glasampulle, Glasvial, Karpule oder Fertigspritze.

12. Gebrauchsfertige Infusionslösung, erhältlich durch Verdünnen der Zusammensetzung nach einem der Ansprüche 1 bis 10 mit einer geeigneten Trägerlösung, insbesondere mit isotonisierter, wässriger Lösung, vorzugsweise mit physiologischer Kochsalzlösung, wässriger Glucoselösung oder isotonen Elektrolyt-Infusionslösungen, beispielsweise Ringerlösung oder Ringer-Lactat-Lösung, insbesondere in einem Verhältnis von 1:10 bis 1:250, vorzugsweise 1:50 zur Verwendung in der Behandlung der peripheren arteriellen Verschlusskrankheit (pAVK) mittels intraarterieller (i.a.) Infusion.

13. Infusionslösung zur Anwendung nach Anspruch 12, wobei Alprostadil in einer Konzentration von 0,04 bis 2 µg/ml, vorzugsweise 0,05 bis 1 µg/ml, insbesondere 0,08 bis 0,8 µg/ml, enthalten ist.

14. Infusionslösung zur Anwendung nach Anspruch 12 oder 13, zur Verwendung in der Behandlung der peripheren arteriellen Verschlusskrankheit (pAVK) im Stadium IIb, III und/oder IV gemäß der Klassifikation von Fontaine.

15. Infusionslösung zur Anwendung nach einem der Ansprüche 12 bis 14, zur Verwendung für eine Verabreichung in die Beckenarterie(n), Unterschenkelarterie(n), Oberschenkelarterie(n), Kniearterie(n) und/oder Leistenarterie(n),
und/oder
zur Verwendung in einer Gesamtdosis von 5 bis 40 µg, insbesondere 10 bis 20 µg, Alprostadil pro Tag,
und/oder
zur Verwendung einmal täglich.

16. Infusionslösung zur Anwendung nach einem der Ansprüche 12 bis 15, zur Verwendung in einer Verdünnung der Zusammensetzung nach einem der Ansprüche 1 bis 15 von 1 ml des Konzentrats in 50 ml isotonischer Natriumchloridlösung.

17. Infusionslösung zur Anwendung nach einem der Ansprüche 12 bis 16, zur Verwendung mit einer Zufuhrrate entsprechend maximal 0,8 g organisches Lösungsmittel, insbesondere Ethanol, pro Stunde,
und/oder
zur Verwendung mit einer Zufuhrrate von 20 - 500 ng Alprostadil pro Minute,
und/oder
zur Verwendung mit einer Infusionsgeschwindigkeit von 0,1 bis 1 ml Infusionslösung/min.

## Claims

1. Liquid pharmaceutical composition in the form of a concentrate for preparing an infusion solution, containing, in particular consisting of, alprostadil (prostaglandin E1) as active substance, dissolved in a pharmaceutically acceptable organic solvent or in a solvent mixture of at least one pharmaceutically acceptable organic solvent and water, for use in the treatment of peripheral arterial occlusive disease (PAOD) by means of intra-arterial (IA) infusion.

2. Composition for use according to claim 1, for use in the treatment of peripheral arterial occlusive disease (PAOD) in stage IIb, III and/or IV according to the Fontaine classification.

3. Composition for use according to any of claims 1 or 2, for use for administration to the pelvic artery/arteries, lower leg artery/arteries, femoral artery/arteries, knee artery/arteries and/or inguinal artery/arteries.

4. Composition for use according to any of claims 1 to 3, for use once a day, in a total dose of 5 to 40 µg, in particular 10 to 20 µg, of alprostadil per day
and/or
for use at a feed rate corresponding to a maximum of 0.8 g of organic solvent, in particular ethanol, per hour.

5. Composition for use according to any of claims 1 to 4, **characterised in that** the proportion of the organic solvent in the solvent mixture is at least 1 wt.% based on the total amount of the solvent mixture, and/or that the proportion of the organic solvent in the solvent mixture is at least 40 wt.% or at least 80 wt.% based on the total amount of the solvent mixture.

6. Composition for use according to any of claims 1 to 5, **characterised in that** the total water content in the solvent mixture, in particular in a mixture with anhydrous ethanol and/or ethanol 96% as solvent, is in the range from 10 to 60 wt.% H₂O, preferably from 20 to 60 wt.% H₂O, in particular from 45 to 55 wt.% H₂O, preferably 50 wt.% H₂O, based on the total amount of the solvent mixture.

7. Composition for use according to any of claims 1 to 6, **characterised in that** the, in particular any, organic solvent is, in particular completely, miscible with water or that the solvent mixture of the at least one organic solvent and water is a true single-phase solution.

8. Composition for use according to any of claims 1 to 7, **characterised in that** the organic solvent is selected from the group consisting of propylene glycol, 1,3 butanediol, ethyl acetate, dimethyl isosorbide, ethanol 96% Ph Eur (95.1-96.9% (v/v)) or anhydrous ethanol Ph Eur (min. 99.5 % (v/v)) or absolute ethanol or mixtures thereof, wherein ethanol 96% Ph Eur (95.1-96.9% (v/v)) and/or anhydrous ethanol Ph Eur (min. 99.5% (v/v)) or absolute ethanol are most preferred as organic solvent.

9. Composition for use according to any of claims 1 to 8, **characterised in that** the composition is free from cyclodextrins, in particular free from α-cyclodextrin and/or β-cyclodextrin,
and/or
that the composition is free from further constituents, excipients or additives, in particular free from salts and/or isotonic additives,
and/or
that alprostadil is contained in a concentration of 1 -1000 µg/ml, preferably 5-750 µg/ml, in particular 20-500 µg/ml.

10. Composition for use according to any of claims 1 to 9, **characterised in that** it is a concentrate for preparing a ready-to-use infusion solution for parenteral administration, in particular by means of a step of dilution with a suitable carrier solution, or is prepared as a concentrate for further preparation of a diluted, ready-to-use infusion solution for parenteral administration and/or
that it is filled into glass ampoules, glass vials, carpules or ready-to-use syringes.

11. Kit-of-parts, containing the composition for use according to any of claims 1 to 10, contained in a sealed glass ampoule, glass vial, carpule or ready-to-use syringe.

12. Ready-to-use infusion solution obtainable by diluting the composition according to any of claims 1 to 10 with a suitable carrier solution, in particular with isotonised, aqueous solution, preferably with physiological saline solution, aqueous glucose solution or isotonic electrolyte infusion solutions, for example Ringer's solution or Ringer's lactate solution, in particular in a ratio of 1:10 to 1:250, preferably 1:50, for use in the treatment of peripheral arterial occlusive disease (PAOD) by means of intra-arterial (IA) infusion.

13. Infusion solution for use according to claim 12, wherein alprostadil is contained in a concentration of 0.04 to 2 µg/ml, preferably 0.05 to 1 µg/ml, in particular 0.08 to 0.8 µg/ml.

14. Infusion solution for use according to claim 12 or 13, for use in the treatment of peripheral arterial occlusive disease (PAOD) in stage IIb, III and/or IV according to the Fontaine classification.

15. Infusion solution for use according to any of claims 12 to 14, for use for administration into the pelvic artery/arteries, lower leg artery/arteries, femoral artery/arteries, knee artery/arteries and/or inguinal artery/arteries,
and/or
for use in a total dose of 5 to 40 µg, in particular 10 to 20 µg, of alprostadil per day and/or
for use once a day.

16. Infusion solution for use according to any of claims 12 to 15, for use in a dilution of the composition according to any of claims 1 to 15 of 1 ml of the concentrate in 50 ml isotonic sodium chloride solution.

17. Infusion solution for use according to any of claims 12 to 16, for use at a feed rate corresponding to a maximum of 0.8 g of organic solvent, in particular ethanol, per hour,
and/or
for use at a feed rate of 20-500 ng alprostadil per minute,
and/or
for use at an infusion rate of 0.1 to 1 ml infusion solution/min.

## Revendications

1. Composition pharmaceutique liquide sous forme d'un concentré pour la préparation d'une solution pour perfusion, comprenant, en particulier composé de, l'alprostadil (prostaglandine E1) en tant que principe actif, dissous dans un solvant organique pharmaceutiquement acceptable ou dans un mélange de solvants composé d'au moins un solvant organique pharmaceutiquement acceptable et d'eau, destinée à être utilisée dans le traitement de l'artériopathie oblitérante des membres inférieurs (AOMI) par perfusion intra-artérielle (voie IA).

2. Composition destinée à être utilisée selon la revendication 1 destinée à être utilisée dans le traitement de l'artériopathie oblitérante des membres inférieurs (AOMI) au stade IIb, III et/ou IV selon la classification de Fontaine.

3. Composition destinée à être utilisée selon l'une quelconque des revendications 1 ou 2, destinée à être utilisée pour une administration dans l'(les)artère(s) iliaque(s), l'(les)artère(s) iliaco-fémorale(s), l'(les)artère(s) fémorale(s), l'(les)artère(s) poplitée(s) et/ou l'(les)artère(s) de l'aine.

4. Composition destinée à être utilisée selon l'une quelconque des revendications 1 à 3, destinée à être utilisée une fois par jour, en une dose totale allant de 5 à 40 µg, en particulier de 10 à 20 µg, d'alprostadil par jour et/ou destinée à être utilisée avec un débit d'administration, correspondant à maximum 0,8 g de solvant organique, en particulier d'éthanol, par heure.

5. Composition destinée à être utilisée selon l'une quelconque des revendications 1 à 4, **caractérisée en ce que** la teneur en solvant organique dans le mélange de solvants est d'au moins 1 % en poids par rapport à la quantité totale du mélange de solvants et/ou **en ce que** la teneur en solvant organique dans le mélange de solvants est d'au moins 40 % en poids ou d'au moins 80 % en poids par rapport à la quantité totale du mélange de solvants.

6. Composition destinée à être utilisée selon l'une quelconque des revendications 1 à 5, **caractérisée en ce que** la teneur en eau totale dans le mélange de solvants, en particulier dans un mélange d'éthanol anhydre et/ou d'éthanol à 96 % en tant que solvant, se situe dans la plage allant de 10 à 60 % en poids d'H₂O, de préférence de 20 à 60 % en poids d'H₂O, en particulier de 45 à 55 % en poids d'H₂O, de préférence à 50 % en poids d'H₂O par rapport à la quantité totale du mélange de solvants.

7. Composition destinée à être utilisée selon l'une quelconque des revendications 1 à 6, **caractérisée en ce que**, en particulier chaque solvant organique est miscible, en particulier en totalité, avec l'eau ou **en ce que** le mélange de solvants composé de l'au moins un solvant organique et d'eau est une véritable solution monophasique.

8. Composition destinée à être utilisée selon l'une quelconque des revendications 1 à 7, **caractérisée en ce que** le solvant organique est choisi parmi le groupe consistant en propylène glycol, butane-1,3-diol, acétate d'éthyle, diméthylisosorbide, éthanol à 96 % Ph. Eur. (95,1 à 96,9 % (V/V)) ou éthanol anhydre Ph. Eur. (min. 99,5 % (V/V)) ou éthanol absolu ou des mélanges de ceux-ci, en tant que solvant organique, l'éthanol à 96 % Ph. Eur. (95,1 à 96,9 % (V/V)) et/ou l'éthanol anhydre Ph. Eur. (min. 99,5 % (V/V)) ou l'éthanol absolu étant particulièrement préférés.

9. Composition destinée à être utilisée selon l'une quelconque des revendications 1 à 8, **caractérisée en ce que** la composition est exempte de cyclodextrines, en particulier exempte de α-cyclodextrines et/ou β-cyclodextrines et/ou **en ce que** la composition est exempte d'autres constituants, excipients ou additifs, en particulier est exempte de sels et/ou d'additifs isotonisants et/ou **en ce que** l'alprostadil est contenu en une concentration de 1 à 1000 µg/ml, de préférence de 5 à 750 µg/ml, en particulier de 20 à 500 µg/ml.

10. Composition destinée à être utilisée selon l'une quelconque des revendications 1 à 9, **caractérisée en ce que** celle-ci est un concentré pour la préparation d'une solution pour perfusion prête à l'emploi destinée à l'administration par voie parentérale, en particulier par une étape de dilution avec une solution support appropriée ou est réalisée en tant que concentré pour la préparation ultérieure d'une solution pour perfusion diluée, prête à l'emploi destinée à l'administration par voie parentérale et/ou **en ce que** celle-ci est conditionnée dans des ampoules en verre, des flacons en verre, des ampoules ou des seringues préremplies.

11. Trousse d'éléments comprenant la composition destinée à être utilisée selon l'une quelconque des revendications 1 à 10, contenue dans une ampoule en verre, flacon en verre, ampoule ou seringue préremplie fermée.

12. Solution pour perfusion prête à l'emploi, pouvant être obtenue par dilution de la composition selon l'une quelconque des revendications 1 à 10 avec une solution support appropriée, en particulier avec une solution aqueuse isotonique, de préférence avec une solution saline physiologique, une solution de glucose aqueuse ou des solutions pour perfusion électrolytiques et isotoniques, par exemple une solution Ringer ou une solution Ringer Lactate, en particulier selon un rapport allant de 1/10 à 1/250, de préférence de 1/50 destinée à être utilisée dans le traitement de l'artériopathie oblitérante des membres inférieurs (AOMI) par perfusion intra-artérielle (voie IA).

13. Solution pour perfusion destinée à être utilisée selon la revendication 12, l'alprostadil étant contenu en une concentration allant de 0,04 à 2 µg/ml, de préférence de 0,05 à 1 µg/ml, en particulier de 0,08 à 0,8 µg/ml.

14. Solution pour perfusion destinée à être utilisée selon la revendication 12 ou 13, destinée à être utilisée dans le traitement de l'artériopathie oblitérante des membres inférieurs (AOMI) au stade IIb, III et/ou IV selon la classification de Fontaine.

15. Solution pour perfusion destinée à être utilisée selon l'une quelconque des revendications 12 à 14, destinée à être utilisée pour une administration dans l'(les)artère(s) iliaque(s), l'(les)artère(s) iliaco-fémorale(s), l'(les)artère(s) fémorale(s), l'(les)artère(s) poplitée(s) et/ou l'(les)artère(s) de l'aine et/ou destinée à être utilisée en une dose totale allant de 5 à 40 µg, en particulier de 10 à 20 µg, d'alprostadil par jour, et/ou destinée à être utilisée une fois par jour.

16. Solution pour perfusion destinée à être utilisée selon l'une quelconque des revendications 12 à 15, destinée à être utilisée à une dilution de la composition selon l'une quelconque des revendications 1 à 15 de 1 ml du concentré dans 50 ml de solution de chlorure de sodium isotonique.

17. Solution pour perfusion destinée à être utilisée selon l'une quelconque des revendications 12 à 16, destinée à être utilisée avec un débit d'administration correspondant à maximum 0,8 g de solvant organique, en particulier l'éthanol, par heure, et/ou destinée à être utilisée avec un débit d'administration de 20 à 500 ng d'alprostadil par minute, et/ou destinée à être utilisée avec une vitesse de perfusion de 0,1 à 1 ml de solution pour perfusion/min.
